Europäisches Patentamt

European Patent Office

Office européen des brevets

(11) Veröffentlichungsnummer: **0 350 442**
**A1**

## (12) EUROPÄISCHE PATENTANMELDUNG

(21) Anmeldenummer: **89810456.7**

(22) Anmeldetag: **13.06.89**

(51) Int. Cl.$^5$: **A 61 F 13/15**

(30) Priorität: **27.06.88 CH 2445/88**

(43) Veröffentlichungstag der Anmeldung:
**10.01.90 Patentblatt 90/02**

(84) Benannte Vertragsstaaten:
**CH DE FR GB IT LI**

(71) Anmelder: **Leone, Demetrio**
**Veilchenweg 17**
**CH-8437 Zurzach (CH)**

(72) Erfinder: **Leone, Demetrio**
**Veilchenweg 17**
**CH-8437 Zurzach (CH)**

(74) Vertreter: **Breiter, Heinz**
**Patentanwalt H. Breiter AG Wartstrasse 4 Postfach 1163**
**CH-8401 Winterthur (CH)**

(54) **Hygienischer, saugfähiger Gegenstand, insbesondere Windel.**

(57) Der hygienische, saugfähige Gegenstand besteht im wesentlichen aus einer äusseren schützenden Schicht (1), einem Saugkissen (3) und einer körpernahen Schicht (4). Im zentralen Bereich, zwischen der äusseren schützenden Schicht (1) und dem Saugkissen (3), ist eine undurchlässige Kunststoffschicht (2) mit wenigstens längsseitig kleineren Flächenabmessungen als diejenigen des Saugkissens (3) eingelegt und/oder eingeklebt, insbesondere mit einem thermoplastischen Schmelzkleber.

FIG.1

EP 0 350 442 A1

## Beschreibung

### Hygienischer, saugfähiger Gegenstand, insbesondere Windel

Die vorliegende Erfindung bezieht sich auf einen hygienischen, saugfähigen Gegenstand, insbesondere auf eine Windel mit einer äusseren schützenden Schicht, einem Saugkissen und einer körpernahen Schicht.

Solche Gegenstände, insbesondere Windeln für Kleinkinder oder für kranke Erwachsene, bzw. auch Damenbinden, sind schon lange bekannt. Als Nässesperre wird meistens eine äussere schützende und tragende Kunststoff-Folie, z.B. aus einem Polyester, verwendet. Dies hat jedoch mehrere Nachteile. Zuerst muss man eine verhältnismässig grosse Fläche dieser Folie ansetzen. Ein weiterer Nachteil besteht darin, dass diese Folie bei Bewegungen knisternde Geräuche verursacht. Es werden auch Materialien verwendet, vor allem chemisch gereinigte Zellulose, die in relativ dicken Schichten, insbesondere für das Saugkissen, angesetzt werden. Somit sind die erwähnten saugfähigen Gegenstände auch verhältnismässig schwer und dick, so dass sie unnötig viel Volumen beanspruchen und für den Transport, beim Verkauf und beim Käufer ungünstig sind.

Die Aufgabe der vorliegenden Erfindung besteht darin, einen hygienischen, saugfähigen Gegenstand, insbesondere eine Windel der eingangs genannten Art zu schaffen, welcher eine günstigere Verteilung der Materialien aufweist, dünn und leicht ist sowie bei genügender Festigkeit gleichzeitig auch ausreichend absorbiert. Weiter sollen knisternde Geräusche des Gegenstandes bei der Bewegung der tragenden Person vermindert werden.

Die Aufgabe wird erfindungsgemäss dadurch gelöst, dass im zentralen Bereich, zwischen der äusseren schützenden Schicht und dem Saugkissen, eine undurchlässige Kunststoffschicht mit wenigstens längsseitig kleineren Flächenabmessungen als diejenigen des Saugkissens eingelegt und/oder eingeklebt ist.

Der Vorteil der erfindungsgemässen Ausbildung ist darin zu sehen, dass die undurchlässige Kunststoffschicht nur in verhältnismässig engen Streifen unter dem Saugkissen angeordnet ist. Somit wird nicht nur Material gespart, es werden auch unangenehme Geräusche bei Bewegung der diesen Gegenstand tragenden Person wesentlich vermindert. Diese verkleinerte Nässesperre unterstützt die Saugeigenschaften des Saugkissens dennoch ausreichend.

Das thermische Einkleben (hot melt) mittels eines Schmelzklebers hat sich gut bewährt. Diese an sich bekannte Art der Befestigungsmethode ist bei der Herstellung des vorliegenden Gegenstands allgemein verwendbar. So kann z.B. zweckmässig die undurchlässige Kunststoffschicht auf die innere Fläche der äusseren schützenden Schicht mittels eines thermoplastischen Schmelzklebers aufgebracht sein.

Bevorzugt sind die äussere schützende Schicht und die körpernahe Schicht als Vlies ausgebildet. Diese Schichten weisen bei richtiger Dimensionierung eine ausreichende Festigkeit auf. Dabei ist es zweckmässig, das Vlies der äusseren schützenden Schicht zwischen 15 - 25 g/m², beispielsweise 21 - 22 g/m², zu wählen.

Es ist vorteilhaft, wenn das Saugkissen aus einem Vlies kombiniert mit einem chemischen Absorptionsmittel insbesondere auch einem Superabsorber, besteht. Das Vlies besteht zweckmässig aus Kunststoffasern, insbesondere aus Polyäthylen oder einem Polyester, oder aus Textilfasern, insbesondere Baumwolle, oder aus Zellulose bzw. aus einer Kombination von wenigstens zwei der erwähnten Materialien. Vorwiegend werden Kunststoffasern und/oder Zellulose angesetzt.

Superabsorber sind hochsaugaktive Polymere, die ein mehrfaches ihres Gewichts an salzhaltigem Wasser oder Urin binden können und dabei ein Gel bilden, das auch unter Druckeinwirkung stabil bleibt. Diese Retention ist eine besonders wichtige Eigenschaft für Windeln, Einlagen, Binden, Tampons und dergleichen. Die Sauggeschwindigkeit eines Superabsorbers ist hauptsächlich von dessen Korngrösse abhängig, d.h. bei kleineren Körnern ist die gesamte Oberfläche des Superabsorbers grösser und deshalb erfolgt die Absorption oder Gel-Bildung wesentlich schneller. Die Einstellung der Saugfähigkeit einer Saugschicht wird mit unterschiedlichen und/oder verschiedenen Mengen von Superabsorbern erreicht.

Als Superabsorber werden beispielsweise folgende Punkte verwendet:

| Aridall 1125 | von Chemdal, U.S.A. |
| Drytech 510 | von Dow Chemical, U.S.A. |
| PR 9910 S | von Floerger, U.S.A. |
| FAVOR 922 SK | von Stockhausen, BRD |

Die undurchlässige Kunststoffschicht besteht vorteilhaft aus Polyäthylen oder Polyester, was auch die thermische Verwendung als Schmelzkleber erleichtert.

Wenigstens im Bereich der vier Ecken sind vorzugsweise klebende Befestigungsbänder angeordnet, welche zweckmässig von den Längsseiten abkragen. Die Befestigungsbänder können auch paarweise einstückig ausgebildet sein.

Das klebende Befestigungsband kann auch, getrennt von dem saugfähigen Gegenstand, auf eine Spule gewickelt werden. Dieses üblicherweise einseitig klebende Band wird beim Befestigen des erfindungsgemässen Gegenstands auf dem Körper in gewählten Längen von der Spule abgewickelt, abgerissen und an der gewünschten Stelle des saugfähigen Gegenstands als Befestigungsband angeordnet, auch paarweise einstückig.

Auf die Aussenfläche der äusseren schützenden Schicht sind vorzugsweise Kunststoff-Flächen oder -Streifen befestigt oder aufgetragen, die als Halterungen für das Befestigungsband dienen. Das Befestigungsband haftet auf dieser Fläche gut und kann ohne Beschädigung des Materials wieder entfernt werden.

Die Erfindung wird anhand eines in der Zeichnung dargestellten Ausführungsbeispiels näher erläutert. Gleiche Teile sind in beiden Figuren mit denselben Bezugsziffern versehen. Es zeigen schematisch:
- Fig. 1 eine Draufsicht auf eine Windel, und
- Fig. 2 einen Schnitt II-II gemäss Fig. 1.

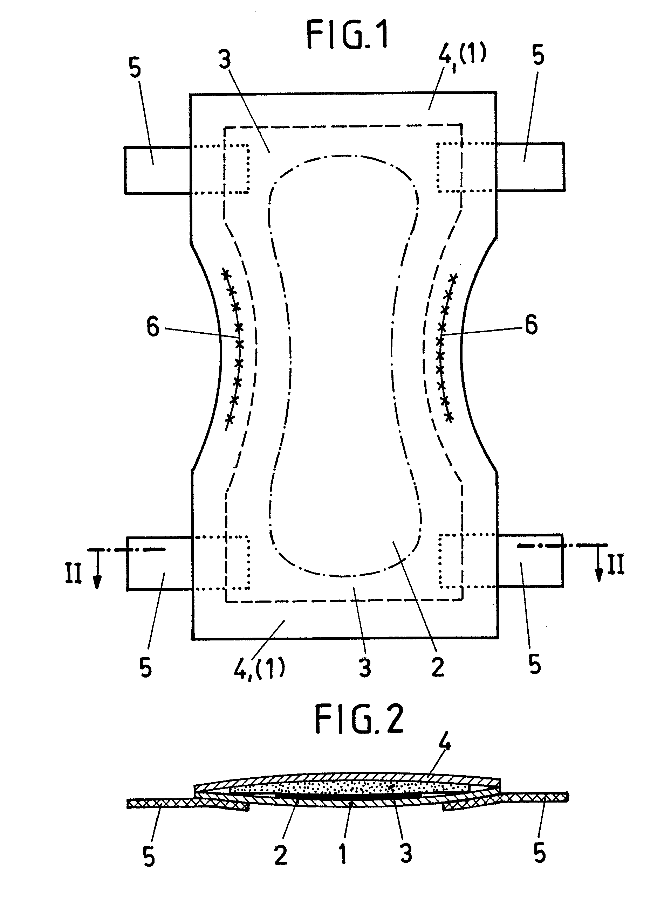

Die äussere schützende Schicht 1 besteht aus einem Vlies, das Zellulosefasern enthält und eine Dichte von 22 g/m² aufweist. Sie weist beidseits längsseitige Einbuchtungen auf. Wie aus Fig. 1 sichtbar, ist die strichpunktiert gezeichnete, undurchlässige Kunststoffschicht 2 aus einer Polyesterfolie nur als verhältnismässig enger Streifen im Mittelbereich der Windel angeordnet. Die Kunststoffschicht 2 ist mit der äusseren schützenden Schicht 1 thermisch verklebt und sowohl längsseitig schmaler als auch stirnseitig verkürzt ausgebildet. Auf der undurchlässigen Kunststoffschicht 2 befindet sich ein gestrichelt gezeichnetes Saugkissen 3, das wesentlich grösser ist als die undurchlässige Kunststoffschicht 2.

Die Windel ist mit einer körpernahen Schicht 4 verschlossen, die gleiche Abmessungen wie die äussere schützende Schicht 1 aufweist und mit dieser entlang des Umfangs verbunden ist.

Vier Befestigungsbänder 5 können wegen der besseren Uebersichtlichkeit gefärbt sein, z.B. blau. Sie bestehen aus einer einseitig oder beidseitig klebenden Kunststoff-Folie, z.B. einer Polyesterfolie.

Mit Linien, die zusätzlich mit Kreuzchen versehen sind, sind abdichtende Gummifasern 6 angedeutet.

Die Windel kann in bekannter Weise verwendet werden. Sie wird in der gewünschten Lage mittels der Befestigungsbänder 5 am Körper fixiert. Nach einer nicht dargestellten Variante können die Befestigungsbänder 5 paarweise einstückig ausgebildet sein.

Die äussere Fläche der äusseren schützenden Schicht 1 kann zusätzlich mit Kunststoff-Streifen versehen sein, die das Befestigen und Entfernen der klebenden Befestigungsbänder 5 erleichtern.

Schliesslich können für die Windel anstelle der Befestigungsbänder 5, Netzhöschen, die über die Windel angezogen werden, als Befestigungsmittel dienen.

Die im Saugkissen 3 enthaltenen absorbierenden Produkte sind, wie erwähnt, handelsüblich. Diese Erzeugnisse weisen sehr kurze Fasern auf, sind chemisch stabil, umweltfreundlich und leicht verarbeitbar.

## Patentansprüche

1. Hygienischer, saugfähiger Gegenstand, insbesondere Windel mit einer äusseren schützenden Schicht (1), einem Saugkissen (3) und einer körpernahen Schicht (4), dadurch gekennzeichnet, dass im zentralen Bereich, zwischen der äusseren schützenden Schicht (1) und dem Saugkissen (3), eine undurchlässige Kunststoffschicht (2) mit wenigstens längsseitig kleineren Flächenabmessungen als diejenigen des Saugkissens (3) eingelegt und/oder eingeklebt ist.

2. Saugfähiger Gegenstand nach Anspruch 1, dadurch gekennzeichnet, dass die undurchlässige Kunststoffschicht (2) mittels eines thermoplastischen Schmelzklebers auf die innere Fläche der äusseren schützenden Schicht (1) aufgebracht ist.

3. Saugfähiger Gegenstand nach Anspruch 1 oder 2, dadurch gekennzeichnet, dass die äussere schützende Schicht (1) und/oder die körpernahe Schicht (4) als Vlies ausgebildet ist.

4. Saugfähiger Gegenstand nach einem der Ansprüche 1 - 3, dadurch gekennzeichnet, dass das Saugkissen (3) aus einem Vlies mit einem chemischen Absorber, insbesondere einem Superabsorber, besteht.

5. Saugfähiger Gegenstand nach einem der Ansprüche 1 - 4, dadurch gekennzeichnet, dass das Vlies aus Kunststoffasern, vorzugsweise aus Polyäthylen oder einem Polyester, und/oder aus Textilfasern, vorzugsweise aus Baumwolle, und/oder aus Zellulose besteht.

6. Saugfähiger Gegenstand nach einem der Ansprüche 1 - 5, dadurch gekennzeichnet, dass die undurchlässige Kunst stoffschicht (2) aus Polyäthylen oder einem Polyester besteht.

7. Saugfähiger Gegenstand nach einem der Ansprüche 1 - 6, dadurch gekennzeichnet, dass wenigstens im Bereich der vier Ecken klebende Befestigungsbänder (5) angeordnet sind, welche vorzugsweise von den Längsseiten abkragen.

8. Saugfähiger Gegenstand nach einem der Ansprüche 1 - 7, dadurch gekennzeichnet, dass die Befestigungsbänder (5) paarweise einstückig ausgebildet sind.

9. Saugfähiger Gegenstand nach einem der Ansprüche 1 - 8, dadurch gekennzeichnet, dass auf die äussere schützende Schicht (1) Kunststoff-Flächen oder -Streifen befestigt oder aufgetragen sind, die als Halterungen für die Befestigungsbänder (5) dienen.

10. Saugfähiger Gegenstand nach einem der Ansprüche 1 - 9, dadurch gekennzeichnet, dass im Bereich der Längsmitte, ausserhalb des Saugkissens (3), abdichtende Gummifasern (6) angeordnet sind.

# FIG.1

# FIG.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0059503 (THE PROCTER & GAMBLE COMPANY) <br> * Seiten 14 - 20; Anspruch 1; Figuren 1, 2 * | 1 | A41B13/02 |
| A | | 2-9 | |
| Y | US-A-3592194 (ROBERT C. DUNCAN) <br> * Spalte 7, Zeile 16 - Spalte 8, Zeile 8 * | 1 | |
| A | * Figur 8 * | 2-9 | |
| A | EP-A-0158914 (KIMBERLY-CLARK CORPORATION) | 1-10 | |

RECHERCHIERTE SACHGEBIETE (Int. Cl.4)

A41B
A61F

Der vorliegende Recherchenbericht wurde für alle Patentansprüche erstellt

| Recherchenort | Abschlußdatum der Recherche | Prüfer |
|---|---|---|
| DEN HAAG | 25 SEPTEMBER 1989 | KARIPIDOU C. |